# EUROPEAN PATENT SPECIFICATION

(11) **EP 2 231 708 B1**
(45) Date of publication and mention of the grant of the patent: **08.04.2015**
(21) Application number: 08859792.7
(22) Date of filing: 12.12.2008
(51) Int. Cl.: C07K 16/28, A61K 39/395, A61P 37/06, A61P 17/00, C12N 5/12

(54) **REMEDIES FOR PEMPHIGUS CONTAINING ANTI-FAS LIGAND ANTIBODIES**
HEILMITTEL GEGEN PEMPHIGUS MIT ANTI-FAS-LIGAND-ANTIKÖRPERN
REMÈDES CONTRE LE PEMPHIGUS CONTENANT DES ANTICORPS ANTI-LIGAND FAS

(30) Priority: 12.12.2007 US 13147 P; 09.05.2008 US 51801 P
(43) Date of publication of application: 29.09.2010
(73) Proprietor: PINCell srl, 41100 Modena (IT)
(72) Inventor: PINCELLI, Carlo, I-41049 Sassuolo (IT); MARCONI, Alessandra, I-42100 Reggio Emilia (IT)
(74) Representative: Weiss, Wolfgang
(86) International application number: PCT/EP2008/010597
(87) International publication number: WO 2009/074339

(56) References cited:
- EP-A- 0 957 166
- US-A1- 2005 106 140
- US-B1- 6 946 255
- PUVIANI MARIO ET AL: "Fas ligand in pemphigus sera induces keratinocyte apoptosis through the activation of caspase-8." THE JOURNAL OF INVESTIGATIVE DERMATOLOGY JAN 2003, vol. 120, no. 1, January 2003 (2003-01), pages 164-167, XP002525807 ISSN: 0022-202X
- ARREDONDO JUAN ET AL: "Novel mechanisms of target cell death and survival and of therapeutic action of IVIg in Pemphigus." THE AMERICAN JOURNAL OF PATHOLOGY DEC 2005, vol. 167, no. 6, December 2005 (2005-12), pages 1531-1544, XP002525808 ISSN: 0002-9440 cited in the application
- VIARD I ET AL: "Inhibition of toxic epidermal necrolysis by blockade of CD95 with human intravenous immunoglobulin" SCIENCE, AMERICAN ASSOCIATION FOR THE ADVANCEMENT OF SCIENCE, US, WASHINGTON, DC, vol. 282, no. 5388, 16 October 1998 (1998-10-16), pages 490-493, XP002119663 ISSN: 0036-8075 cited in the application
- WANG X ET AL: "Possible apoptotic mechanism in epidermal cell acantholysis induced by pemphigus vulgaris autoimmunoglobulins." APOPTOSIS : AN INTERNATIONAL JOURNAL ON PROGRAMMED CELL DEATH MAR 2004, vol. 9, no. 2, March 2004 (2004-03), pages 131-143, XP002525809 ISSN: 1360-8185 cited in the application
- JU-WON KWAK ET AL: "A convenient method for epitope competition analysis of two monoclonal antibodies for their antigen binding", JOURNAL OF IMMUNOLOGICAL METHODS, ELSEVIER SCIENCE PUBLISHERS B.V.,AMSTERDAM, NL, vol. 191, no. 1, 10 May 1996 (1996-05-10), pages 49-54, XP004020853, ISSN: 0022-1759, DOI: 10.1016/0022-1759(95)00287-1
- WASEEM N H ET AL: "MONOCLONAL ANTIBODY ANALYSIS OF THE PROLIFERATING CELL NUCLEAR ANTIGEN (PCNA) STRUCTURAL CONSERVATION AND THE DETECTION OF A NUCLEOLAR FORM", JOURNAL OF CELL SCIENCE, CAMBRIDGE UNIVERSITY PRESS, LONDON, GB, vol. 96, no. 1, 1 May 1990 (1990-05-01), pages 121-129, XP000938974, ISSN: 0021-9533
- ANONYMOUS: 'T 1918/06 (Lymphocyte adherence/FRED HUTCHINSON CANCER RESEARCH CENTER) of 10.3.2010', [Online] EPO Retrieved from the Internet: <URL:http://www.epo.org/law-practice/case-l aw-appeals/recent/t061918eu1.html>
- ANONYMOUS: 'Epitope mapping', [Online] www.sigmaaldrich.com
- B. M. REIPERT ET AL: "Variation of anti-Fas antibodies in different lots of intravenous immunoglobulin", VOX SANGUINIS, vol. 94, no. 4, 1 May 2008 (2008-05-01), pages 334-341, XP055049093, ISSN: 0042-9007, DOI: 10.1111/j.1423-0410.2008.001036.x

## Description

The present invention refers to the use of antagonists of human Fas ligand (also named CD95L or Apo1L and hereinafter abbreviated as FasL), more particularly to the use of humanized antibodies against FasL for the prevention and/or treatment of skin diseases associated with keratinocyte acantholysis, particularly for the prevention and/or treatment of pemphigus.

Pemphigus is an autoimmune bullous skin condition characterized by loss of keratinocyte adhesion due to autoantibodies directed against desmosomal proteins (desmogleins, dsg). Pemphigus has a world-wide distribution with an incidence of approximately 1-5 per 100.000 per year, and with a female predominance.

Pemphigus is characterized by loss of adhesion of suprabasal keratinocytes that round up in a process known as acantholysis. Pathogenesis of pemphigus is undergoing a major revision, mostly because, in addition to anti-desmoglein antibodies, a new group of anti-cholinergic receptor antibodies can induce acantholysis (Kalish, 2000). Further, it has been demonstrated that steric hindrance alone cannot account for blister formation upon antigen-antibody binding (Kitajima, 2002). Further, it was shown that desmosome formation is not inhibited by pemphigus autoantibodies (PVIgG) binding to pemphigus, while the desmosomal connections are dissociated 24-36 hrs after treatment with PvIgG. During this time, a series of signal transduction steps triggered by PVIgG take place.

These observations suggest a role for apoptosis in pemphigus. Indeed, pemphigus is a disease due to lack of cell adhesion (Payne AS et al, 1978) and apoptosis can occur in association with cell detachment (Marconi et al, 2004). These concepts are fully confirmed by the detection of apoptosis in lesional pemphigus skin. In particular, acantholytic cells express the major markers of apoptosis (Wang X et al, 2004). More interestingly, TUNEL-labeling nuclei are also found attached to the blister roof, suggesting the presence of apoptotic cells in perilesional skin before detachment. Moreover, apoptotic cells expressing Ig and activated caspase-8 are detected at the edge of the lesion, in areas where no disruption of cell-cell contacts is visible (Wang X et I, 2004). These data definitely demonstrate that in pemphigus, apoptosis takes place in keratinocytes before acantholysis.

Apoptosis plays a fundamental role in the regulation of cellular homeostasis and is involved in many pathophysiologic processes. Apoptosis can follow both cell to cell detachment (Rezgui *et al,* 2000; Bergin *et al,* 2000) and loss of cell-matrix interaction (Giancotti and Ruoslahti, 1999).

Fas (or FasR) is a member of the TNF-receptor superfamily which, upon binding with Fas ligand (FasL), triggers apoptosis in many cell systems (Sharma *et al,* 2000). Intracellular signaling of Fas-FasL-induced apoptosis operates via recruitment of a number of adaptor molecules such as FADD (Fas-associated death domain) and FLICE (FADD-like ICE, caspase 8), which in turn is inhibited by FLIP (FLICE inhibitory protein) (Juo *et al,* 1998). Fas-FasL interaction is involved in the pathomechanisms of several immune-inflammatory and infectious conditions, such as AIDS (Bahr *et al,* 1997) and systemic lupus erythematosus (Kovacs *et al,* 1997). Cutaneous diseases characterized by an implication of Fas-FasL pathway include acute graft versus host disease, toxic epidermal necrolysis and melanoma (Wehrli *et al,* 2000). Further, it was reported that acantholytic-like lesions are observed in cultured keratinocytes treated with both PVIgG and with anti-FasR, while PvIgG induce the clustering of FasR, FasL and caspase-8 on the cell membrane several hours before the formation of the lesions (Wang X et al, 2004). Moreover, it was reported that the caspase-1-like inhibitor significantly blocked the blister formation in a pemphigus mouse model (Li et al, 2006). Taken together, these data indicate that FasL and the extrinsic apoptotic pathway play a critical role in the mechanisms underlying acantholysis.

Whatever the nature of the pemphigus autoantibodies, exposure to PVIgG up-regulates the expression of several pro-apoptotic genes, including FasL, many hours before acantholysis. Moreover, intravenous IgG (IVIgG) prevents PvIgG-induced up-regulation of FasL and apoptosis in keratinocytes. IVIgG also prevents acantholysis and apoptosis in vivo (Arredondo J et al, 2005).

Puviani et al. (J. Investigative Dermatology, Vol. 20, No. 1, Jan. 2003, pp. 164-169) disclose that addition of anti-FasL neutralizing antibodies partially inhibits in vitro pemphigus-sera-induced keratinocyte apoptosis. Puviani et al. do not provide any evidence of an effect on acantholysis or of in vivo efficacy of said antibodies.

Without treatment, the mortality of pemphigus vulgaris approaches 100%. Early systemic therapy is required to control pemphigus, but side effects from systemic therapy are a major complication. Treatment includes administration of corticosteroids, medications containing gold, the antiinflammatory drug dapsone, or medications that suppress the immune system (such as azathioprine, methotrexate, cyclosporin, cyclophosphamide, or mycophenolate mofetil). The most common treatment for pemphigus is nowadays steroids which need to be administered for life and can cause severe side effects. Most pemphigus patients die by these side effects. Some antibiotics are also effective, particularly minocycline and doxycycline. Intravenous immunoglobulin (IVIg) is occasionally used. Plasmapheresis is a process whereby antibody-containing plasma is removed from the blood and replaced with intravenous fluids or donated plasma. Plasmapheresis may be used in addition to the systemic medications to reduce the amount of antibodies in the bloodstream. A number of new molecules are also under development: mycophenolate mofetil, PI-0824, PRTX-100, anti-CD20 are immunesuppressive drugs which act on T and B cells.

The current mortality rate still ranges from 5 to 25 %; infection is the most frequent cause of death and long-term immunosuppressive therapy (mainly corticosteroid) is one of the significant factors still provoking a high mortality rate. Immunoglobulin can produce some short-term improvement, but does not seem to induce lasting remissions, and its cost makes it impractical for long-term use.

There are several caveats also in the use of plasmapheresis. Patients suppressed with prednisone or others immunosuppressive agents and receiving plasmapheresis, are at higher risk of sudden death from sepsis. In addition, the treatment is very expensive, and a 2-week hospitalization period is necessary to administer the therapy. Therefore, because of the risk of sudden death from sepsis and the high cost, plasmaphaeresis is indicated only in the most refractory cases where the patient is clearly at risk of dying from the disease itself.

It is an object of the present invention to provide a therapeutic agent for pemphigus. The development of a new drug which blocks FasL would allow to completely prevent cell detachment and the formation of the skin lesion.

In general, the present invention refers to the treatment of a skin disease associated with keratinocyte acantholysis by administering a FasL antagonist. FasL antagonists may be selected from anti FasL antibodies, particularly humanized or human anti FasL antibodies, nucleic acid effector molecules of Fas expression such as antisense molecules or molecules capable of RNA interference such as siRNA molecules, soluble Fas receptor molecules, antagonistic FasL muteins, and low molecular weight chemical compounds inhibiting the Fas-FasL interaction. FasL antagonists prevent keratinocyte apoptosis and subsequent cell-cell detachment (acantholysis). Thus, FasL antagonists are particularly suitable for the prevention and/or treatment of pemphigus, e.g. for the prevention and/or treatment of mucocutaneous pemphigus.

The present invention relates to a medicament containing at least one compound inhibiting the biological effects of FasL. The expression "compound inhibiting the biological effects of FasL" used herein relates to all the compounds which can fully or at least substantially inhibit or neutralize the biological effects of FasL. For example, the inhibitory or neutralizing effect may be based on suppressing the binding of FasL to its natural receptor and therefore the thus caused signal transmissions. This can be achieved e.g. by using antibodies binding to FasL per se or soluble receptors mimicking Fas or antagonistic FasL muteins, thus blocking the binding of FasL to the cellular receptors. Interfering with Fas or FasL expression by siRNA will block Fas/FasL system.

FasL antagonist therapy is either a monotherapy or be given in combination with other medicaments suitable for the treatment of pemphigus or other skin diseases, particularly as described above. For example, a combination therapy of FasL antagonists and steroids might allow a drastic reduction of the steroid doses.

In a preferred embodiment of the present invention, there is provided a therapeutic agent for pemphigus, comprising an antibody against a human Fas ligand, or an active fragment thereof as an active ingredient. The antibody is preferably a chimeric, humanized or human anti-FasL antibody or an antigen-binding fragment or derivative, e.g. a recombinant single chain antibody. If desired, the antibody may be conjugated to effector molecules, e.g. cytostatic, cytotoxic and/or radioactive compounds.

Preferred humanized antibodies suitable for the treatment of skin disease associated with keratinocyte acantholysis, in particular pemphigus according to the present invention, are described in WO 1997/002290A1 ("Anti-Fas ligand antibodies and assay method using the same antibody") or in WO 1998/010070 A1 ("Humanized immunoglobulin reacting specifically with Fas Ligand or active fragments thereof and region inducing apoptosis originating in Fas Ligand humanized antibodies").

Further preferred humanized antibodies suitable for the treatment of skin disease associated with keratinocyte acantholysis, in particular pemphigus, according to the present invention, are described in US 7,262,277 ("Antagonistic Anti-hFas ligand human antibodies and fragments thereof).

Human or humanized antibodies have at least three potential advantages over mouse and in some cases chimeric antibodies for use in human therapy: 1) because the effector portion is human, it may interact better with the other parts of the human system; 2) the human immune system should not recognize the framework or C region of the humanized antibody as foreign, and therefore the antibody response against such an injected antibody should be less than against a totally foreign mouse antibody or a partially foreign chimeric antibody; 3) injected humanized antibodies will presumably have a half-life more like that of naturally occurring human antibodies, allowing smaller and less frequent doses to be given.

Further preferred FasL antagonists are soluble FasR molecules comprising the extracellular soluble part of the Fas receptor or modified antagonistic FasL molecules which have a competitive or non-competitive antagonistic activity. These molecules inhibit FasL/FasR interactions in that FasL binds to the soluble receptor analogue or the antagonistic FasL molecule binds to the natural receptor thereby reducing or fully eliminating the binding of biologically active FasL to the natural receptor. During treatment with siRNA, an analysis of Fas and/or FasL protein or RNA levels can be used to determine treatment type and the course of therapy in treating a subject. Monitoring of Fas and/or FasL protein or RNA levels can be used to predict treatment outcome and to determine the efficacy of compounds and compositions that modulate the level and/or activity of certain Fas and/or FasL proteins associated with a trait, condition, or disease.

In an especially preferred aspect, the present invention refers to an antibody selected from
(i) a monoclonal antibody or an antigen-binding fragment thereof specific for human Fas ligand protein (FasL), wherein said monoclonal antibody comprises at least one heavy chain variable region and at least one light chain variable region, wherein the amino acid sequences of the complementary determining region (CDRs) of the heavy chain are:
   (a₁) CDR H1: Asn Tyr Trp Ile Gly (SEQ ID NO:1),

(b1) CDR H2: Tyr Leu Tyr Pro Gly Gly Leu Tyr Thr Asn Tyr Asn Glu

Lys Phe Lys Gly (SEQ ID NO:2),

   (c₁) CDR H3: Tyr Arg Asp Tyr Asp Tyr Ala Met Asp Tyr (SEQ ID NO:3)
and the amino acid sequences of the complementary determining regions (CDRs) of the light chain are

(a₂) CDR L1: Lys Ser Thr Lys Ser Leu Leu Asn Ser Asp Gly Phe
Thr Tyr Leu Gly (SEQ ID NO:4),

(b₂) CDR L2: Leu Val Ser Asn Arg Phe Ser (SEQ ID NO:5),
(c₂) CDR L3: Phe Gln Ser Asn Tyr Leu Pro Leu Thr (SEQ ID NO:6)
for use in the prevention and/or treatment of pemphigus.

Also described herein is an antibody or an antigen-binding fragment thereof which recognizes the same epitope on human FasL as the antibody (i) described above, for the manufacture of a medicament for the prevention and/or treatment of a skin disease associated with keratinocyte acanatholysis, particularly of pemphigus.

The present specification further discloses the use of
(i) a monoclonal antibody or an antibody-binding fragment thereof specific for human Fas ligand protein (FasL), wherein the monoclonal antibody is produced by the hybridoma cell under Accession No. FERM BP-5045 or an antibody or antibody fragment derived therefrom, or
(ii) a monoclonal antibody or an antigen-binding fragment thereof which recognizes the same epitope of human FasL as the antibody of (i)
for the manufacture of a medicament for the prevention and/or treatment of a skin disease associated with keratinocyte acantholysis, particularly of pemphigus.

In a further aspect, the present invention refers to an antibody selected from
(i) a monoclonal antibody or an antigen-binding fragment thereof specific for human Fas ligand protein (FasL), wherein said monoclonal antibody comprises at least one heavy chain variable region and at least one light chain variable region, wherein the amino acid sequences of the complementary determining region (CDRs) of the heavy chain are:
   (a₁) CDR H1: Glu Tyr Pro Met His (SEQ ID NO:7),

(b1) CDR H2: Met Ile Tyr Thr Asp Thr Gly Glu Pro Ser Tyr Ala Glu
Glu Phe Lys Gly (SEQ ID NO:8),

   (c₁) CDR H3: Phe Tyr Trp Asp Tyr Phe Asp Tyr (SEQ ID NO:9)
and the amino acid sequences of the complementary determining regions (CDRs) of the light chain are
(a₂) CDR L1: Arg Ala Ser Gin Asp Ile Ser Asn Tyr Leu Asn (SEQ ID NO:10),
(b₂) CDR L2:Tyr Thr Ser Arg Leu His Ser (SEQ ID NO:11),
(c₂) CDR L3:Gln Gln Gly Ser Thr Leu Pro Trp Thr (SEQ ID NO:12)
for use in the prevention and/or treatment of pemphigus.

Also described herein is an antibody or an antigen-binding fragment thereof which recognizes the same epitope on human FasL as the antibody (i) described above, for the manufacture of a medicament for the prevention and/or treatment of a skin disease associated with keratinocyte acanatholysis, particularly of pemphigus.

The present specification further discloses the use of
(i) a monoclonal antibody or an antibody-binding fragment thereof specific for human Fas ligand protein (FasL), wherein the monoclonal antibody is produced by the hybridoma cell under Accession No. FERM BP-5533, FERM BP-5534 and/or FERM BP-5535 or an antibody or antibody fragment derived therefrom, or
(ii) a monoclonal antibody or an antigen-binding fragment thereof which recognizes the same epitope of human FasL as the antibody of (i)
for the manufacture of a medicament for the prevention and/or treatment of a skin disease associated with keratinocyte acantholysis, particularly of pemphigus.

The present specification moreover discloses
the use of anti-FasL human antibodies, or antigen-binding portions thereof, comprising a light chain variable region and/or a heavy chain variable region as described in US 7,262,277,

In particular, anti-FasL human antibodies suitable for the treatment of skin disease associated with keratinocyte acantholysis disclosed herein comprise a light chain variable region comprising a polypeptide with the sequence shown in SEQ ID NO 2 of US 7,262,277 and further comprising a heavy chain variable region comprising a polypeptide with the sequence shown in SEQ ID NO 10 or 18 of US 7,262,277.

More particularly, the specification discloses the use of the anti-hFas human antibody 3E1 and/or 4G11 as described in US 7,262,277 for the manufacture of a medicament for the prevention and/or treatment of skin disease associated with keratinocyte acantholysis, particularly of pemphigus.

In this aspect, the present specification discloses the use of
(i) a monoclonal human antibody or an antigen-binding fragment thereof specific for human Fas ligand protein (FasL), wherein said monoclonal antibody comprises at least one heavy chain variable region and at least one light chain variable region, wherein the amino acid sequences of the complementary determining regions (CDRs) of the heavy chain are:
   (a₁) CDR H1: Arg His Gly Ile Thr (SEQ ID NO: 13) or
   (a₂) CDR H1: Ser His Gly Ile Ser (SEQ ID NO: 14), 
(b₁) CDR H2: Trp Ile Asn Ala Tyr Asn Gly Asn Thr Asn Tyr Ala
Gln Lys Val Gln Gly (SEQ ID NO: 15)
   or

(b₂) CDR H2: Trp Ile Asn Ala Tyr Ser Gly Asn Thr Asn Tyr Ala
Gln Lys Leu Gln Gly (SEQ ID NO: 16),

   (c₁) CDR H3: Glu Thr Met Val Arg Gly Val Pro Leu Asp Tyr (SEQ ID NO: 17) or
   (c₂) CDR H3: Glu Thr Met Val Arg Gly Val Pro Cys Asp Tyr (SEQ ID NO: 18), or
   (d₁) a sequence derived by substituting 1, 2 or 3 amino acids of SEQ ID NOs 13, 14, 15, 16, 17 and/or 18,
   and/or the amino acid sequences of the complementary determining regions (CDRs) of the light chain are
   (a₃) CDR L1: Arg Ala Ser Gln Ser Val Ser Ser Ser Tyr Leu Ala (SEQ ID NO: 19),
   (b₃) CDR L2: Gly Ala Ser Ser Arg Ala Thr (SEQ ID NO: 20),
   (c₃) CDR L3: Gln Gln Tyr Gly Ser Ser Pro Trp Thr (SEQ ID NO: 21) or
   (d₃) a sequence derived by substituting 1, 2 or 3 amino acids of SEQ ID NOs: 19, 20 and/or 21
   or
(ii) an antibody or an antigen-binding fragment thereof which recognizes the same epitope on human FasL as the antibody (i),
for the manufacture of a medicament for the prevention and/or treatment of a skin disease associated with keratinocyte acantholysis, particularly of pemphigus.

Finally, the present specification discloses the use of
(i) a monoclonal antibody or an antibody-binding fragment thereof specific for human Fas ligand protein (FasL), wherein the monoclonal antibody is produced by the hybridoma cell under Accession No. ATCC PTA-4017 and/or ATCC PTA-4018 or an antibody or antibody fragment derived therefrom, or
(ii) a monoclonal antibody or an antigen-binding fragment thereof which recognizes the same epitope of human FasL as the antibody of (i)
for the manufacture of a medicament for the prevention and/or treatment of a skin disease associated with keratinocyte acantholysis, particularly of pemphigus.

The medicament of the present invention may be provided as a pharmaceutical composition together with a pharmaceutically acceptable carrier. Preferably, the pharmaceutical composition is administered by injection or infusion, e.g. intravenously, intraarterially, subcutaneously, intraperitoneally or by other suitable means. The composition may be administered locally or systemically. Preferably, the composition is administered systemically.

Pharmaceutical compositions suitable for use in the invention comprise the active agent in an effective amount to achieve the intended purpose. An effective dose of a medicament of the present invention may be in the range of 0.1 µg to 100 mg, up to a total dose of about 1 g depending upon the route of administration. The pharmaceutical compositions may be administered daily, e.g. once or several times, or every two to four days, every week or once in two weeks. The medicament may be administered in a single treatment cycle consisting of one or several medicament administrations or in several treatment cycles each consisting of one or several medicament administrations. Each treatment cycle may have a duration of one day up to several weeks, months, or even years.

Hence, according to the present invention the medicament may also be used in a combination therapy with at least one further therapy effective against a skin disease associated with keratinocyte acantholysis and in particular against pemphigus. The medicament will be used either alone or in combination with other immunosuppressive drugs, particularly with steroids, in order to reduce their dose and/or to minimize their chronic side effects. When used in combination, the medicament will preferably be administered on a monthly basis. When used alone, the medicament will preferably be administered continuously within a time frame depending on the individual case.

Further, the present invention shall be explained in more detail by the following examples.

### Examples

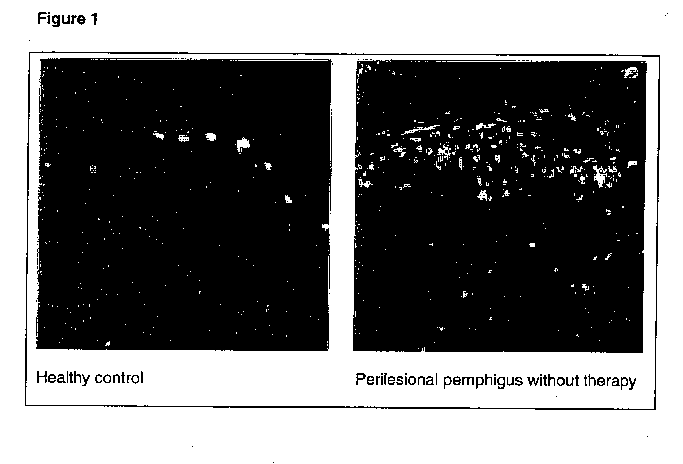

We first evaluated the presence of apoptosis in epidermis from perilesional skin in frozen sections from untreated pemphigus patients by TUNEL staining. Fluorescent specimens were analyzed by confocal scanning laser microscopy. In suprabasal layers from perilesional epidermis most keratinocytes are apoptotic, as compared to normal skin (Fig 1).

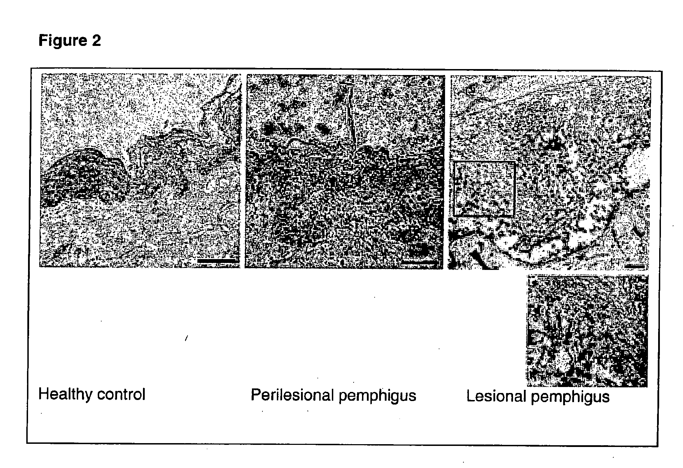

In order to confirm apoptosis in pemphigus lesions we used formalin-fixed and paraffin embedded biopsies and detected the active form of caspase-3. Staining protocol was performed by UltraVision LP Detection System AP Polymer and Fast Red Chromogen (Lab Vision Corporation, CA, USA) according to manufacturer's instruction. Visualization was obtained with Fast Red tablets in naphthol phosphate substrate. In pemphigus samples we found that caspase-3 fragment is located both in the roof and in the floor of the blister, with some cells being positive in perilesional epidermis (Fig 2). This result seems to indicate that keratinocyte cell death occurs before the detachment of keratinocytes leading to acantholysis.

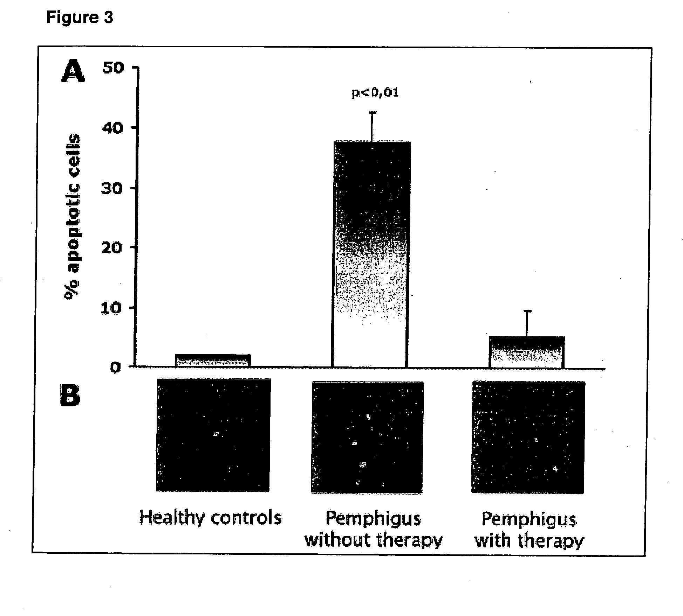

As apoptotic keratinocytes are abundantly expressed in pemphigus, we wanted to explore whether pemphigus sera are capable of inducing apoptosis in normal human keratinocytes. To this purpose, keratinocytes were plated in chamber slides and cultured in serum-free medium (KGM) up to preconfluence. Cells were then cultured in keratinocyte basal medium and treated for 48 h with the addition of 25% serum from either untreated patients or patients treated with systemic corticosteroids. Sera from healthy subjects were used as controls. Apoptosis was evaluated by TUNEL staining in situ. Approximately 100 cells were evaluated, in randomly selected fields, and the percentage of TUNEL-positive cells was counted. Sera from pemphigus but not from healthy subjects or patients undergoing steroid treatment induced apoptosis in human keratinocytes (Fig 3A-B).

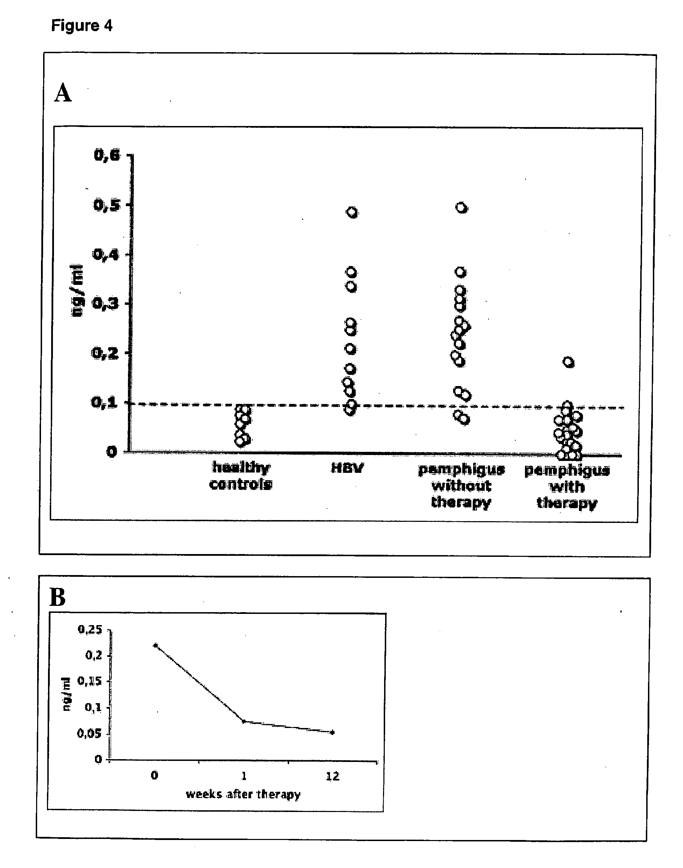

As the Fas/FasL system is implicated in many apoptotic processes also at the skin level (Wehrli *et al,* 2000), we measured FasL levels in sera from pemphigus patients by a two-site enzyme immunoassay (ELISA). Serum concentration was determined by absorbance at 450 nm against recombinant human FasL standard protein. FasL levels were very high in sera from untreated patients and below the limit of detection in sera from patients treated with corticosteroids or in sera from healthy subjects. Sera from HBV patients were used as positive control (Fig 4A). In one patient, FasL levels progressively decreased with systemic steroid therapy (Fig 4B).

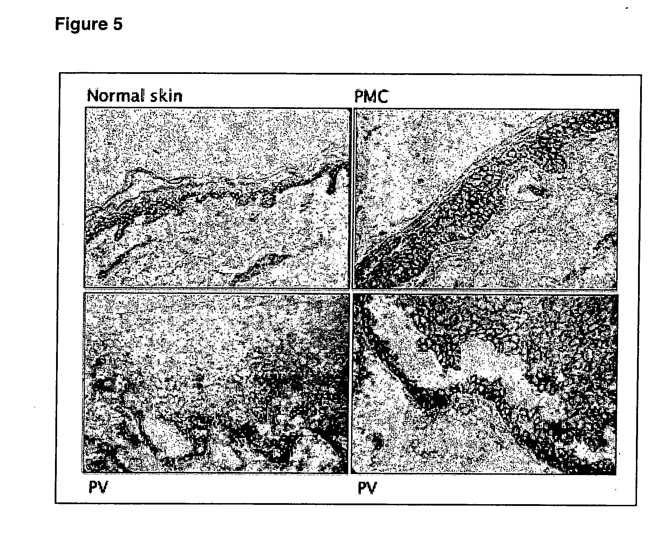

As FasL is contained in high amounts in pemphigus sera, we looked at the expression of its cognate receptor FasR. To this purpose we used formalin-fixed and paraffin embedded biopsies. Staining protocol was performed by UltraVision LP Detection System AP Polymer and Fast Red Chromogen (Lab Vision Corporation, CA, USA) according to manufacturer's instruction. Visualization was obtained with Fast Red tablets in naphthol phosphate substrate. While FasR is expressed only in basal keratinocytes in normal skin, in active pemphigus lesions, FasR is detected both in the basal and in the suprabasal cells. Even more intriguing, in mucocutaneous pemphigus (PMC) FasR seems to be expressed throughout the epidermal layers and even before blister formation (Fig 5).

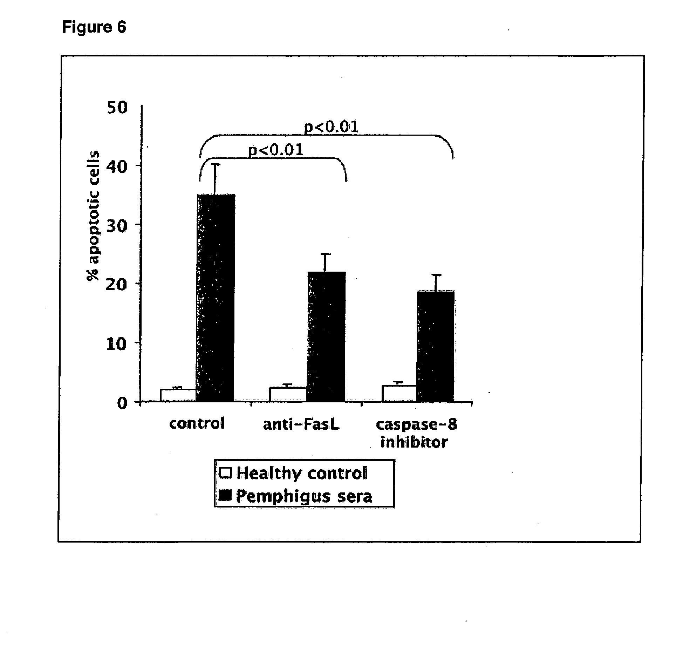

FasL is one of the major triggers of the caspase-8 activated extrinsic apoptotic pathway. Therefore, we wanted to evaluate whether this pathway plays a role in pemphigus apoptosis. To this purpose, patient sera were pretreated with anti-FasL neutralizing antibody or caspase-8 inhibitor, and added to keratinocyte cultures. Keratinocytes were cultured in KGM and treated with pemphigus sera or with sera from untreated patients. Sera were pretreated with anti-FasL neutralizing antibody (2.5 mg per ml for 30 min) or caspase-8 inhibitor Z-IETD-FMK (100 µM for 30 min). Apoptosis was evaluated by TUNEL staining. Addition of anti-FasL neutralizing antibody or caspase-8 inhibitor partially prevented pemphigus sera-induced keratinocyte apoptosis (Fig 6).

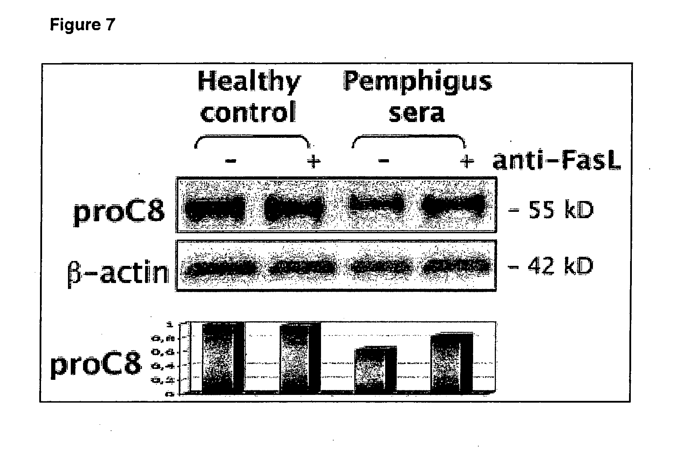

In addition, Keratinocytes were treated as in Fig 6 and provided with either anti-FasL antibody or irrelevant immunoglobulins. Cells were then homogenized in RIPA buffer for Western blotting analysis. Membranes were incubated with anti human caspase-8 or anti-b-actin antibodies. The relative intensity of bands on autoradiograms was quantified by scanning laser densitometry. The results shown that caspase-8 was markedly activated in keratinocytes treated with pemphigus sera, as compared to untreated cells, while caspase cleavage was partially inhibited by pre-treatment with anti-FasL antibody (Fig. 7).

Taken together, these data suggest that pemphigus sera induce keratinocyte apoptosis through the extrinsic apoptotic pathway triggered by the Fas/FasL system.

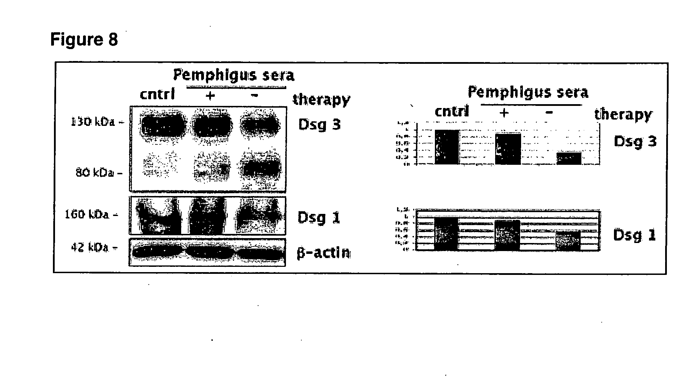

Recent studies have shown that components of the cadherin-catenin adhesion complex in epithelial adherens junctions are targeted by caspases during apoptosis (Weiske et al, 2001). In order to evaluate whether Fas/FasL-induced apoptotic pathway is also responsible for desmosomal separation, we treated for 72 hrs confluent keratinocytes, cultivated in KGM in presence of 1.8 mM CaCl₂, with pemphigus sera with or without therapy. Protein extracts from the culture were analyzed by Western blotting using anti-Dsg1 and anti-Dsg3 antibodies. β-actin was used as internal control. We found that pemphigus sera can cleave Dsg1 and Dsg3. In particular, sera from untreated patients, but not from patients under steroid therapy strikingly cleave Dsg1 and Dsg3. (Fig 8).

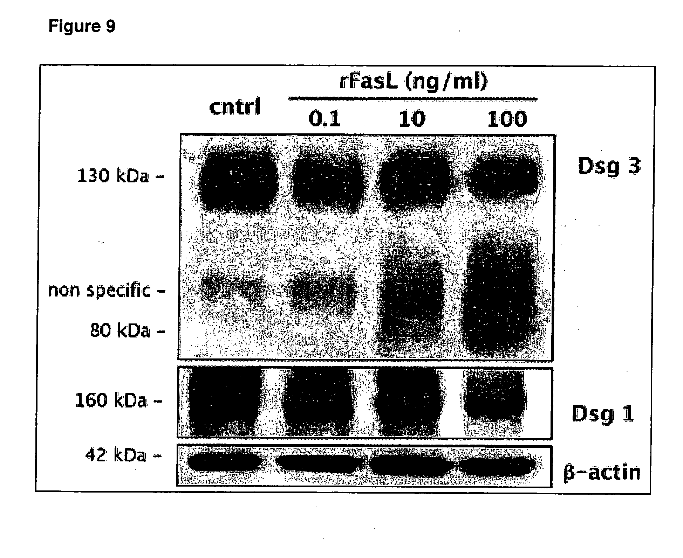

Most importantly, treatment of keratinocytes with increasing amounts of FasL (0.1, 10, 100 ng/ml) for 72 hrs, cleaved dsgs in a dose-dependent manner. Protein extracts from the culture were analyzed by Western blotting using anti-Dsg1 and anti-Dsg3 antibodies. β-actin was used as internal control. These doses are consistent with the ones detected in pemphigus sera (Fig 9).

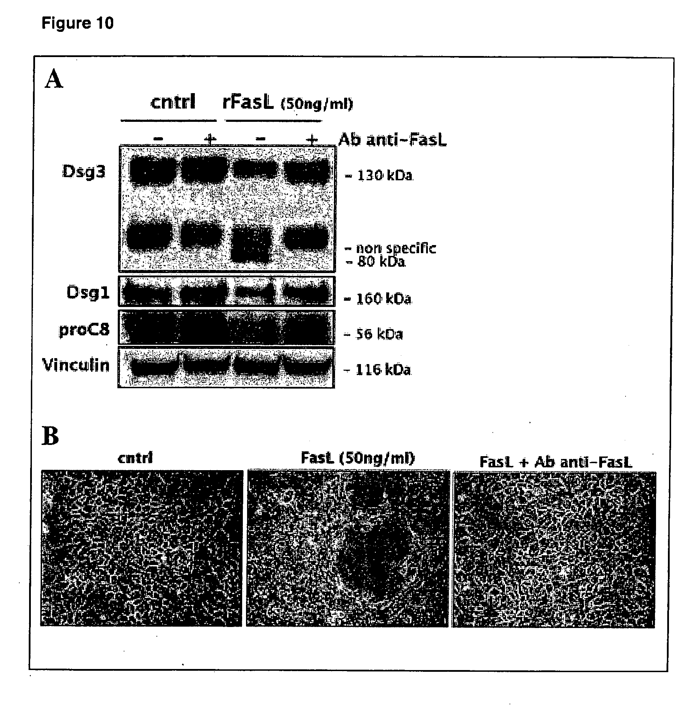

Given that FasL exerts an important role in the pathogenesis of pemphigus, we have tested an anti-FasL antibody (NOK2, antibody produced by the hybridoma cell line NOK2, accession number No. FERM BP-5045). Confluent keratinocytes, cultivated in KGM with 1,8 mM CaCl₂, were treated for 72 hrs with: 1. KGM alone; 2. anti-FasL (NOK2, 15 µg/ml) Ab; 3. FasL (50 ng/ml); 4. FasL + anti-FasL Ab. We present evidence that anti-FasL Ab (NOK2) prevents FasL-induced dsg cleavage. We also show that anti-FasL Ab (NOK2) inhibits caspase-8-induced apoptosis (Fig 10 A). Fig 10B shows that anti-FasL (NOK2) Ab prevents FasL-induced cell-to-cell detachment, i.e. acantholysis.

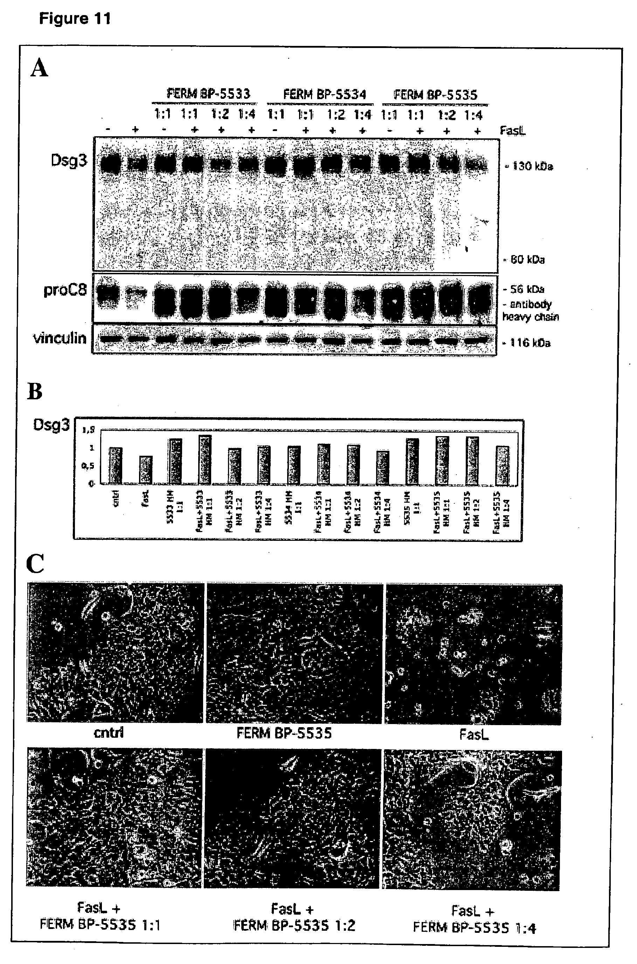

In order to further confirm the central role of FasL, we have used other anti-FasL antibodies (F918-7-3, antibody produced by the hybridoma cell line with accession number No. FERM BP-5533; F918-7-4, antibody produced by the hybridoma cell line with accession number No. FERM BP-5534; F919-9-18, antibody produced by the hybridoma cell line with accession number No. FERM BP-5535). Confluent keratinocytes, cultivated in KGM with 1,8 mM CaCl₂, were treated for 72 hrs with: KGM alone; recombinant FasL (50 ng/ml); hybridoma medium diluted 1:1 in KGM; FasL + hybridoma medium at different dilution in KGM. We present evidence that anti-FasL antibodies prevents FasL-induced dsg3 cleavage in a dose-dependent manner (Fig 11A and Fig 11B), inhibiting caspase-8 induced apoptosis activation (Fig 11 A). Fig 11C shows that the FasL Ab contained in the medium from hybridoma cell line FERM BP-5535 prevents FasL-induced cell-to-cell detachment, i.e. acantholysis.

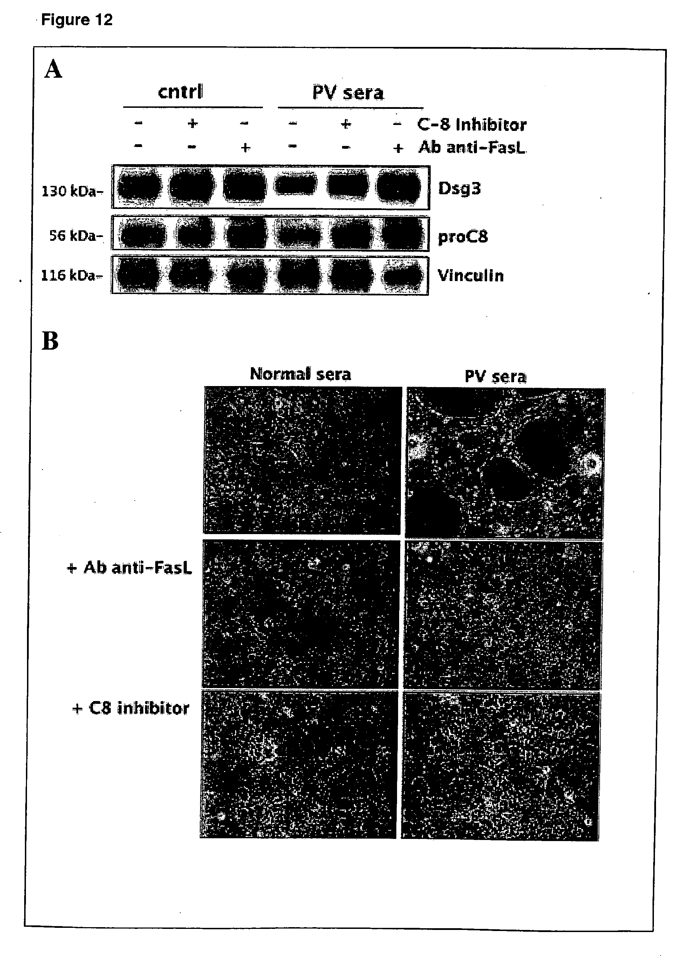

To investigate whether the extrinsic apoptotic pathway is responsible for dsg cleavage, we pretreated confluent keratinocytes with caspase-8 inhibitor Z-IETD-FMK (100 µM for 30 min) or with anti-FasL (NOK2, 15 µg/ml)Ab. Then cells were incubated for 72 hrs with healthy or untreated pemphigus sera. Protein extracts from the culture were analyzed by Western blotting using anti-Dsg3 antibodies and anti-caspase-8 Ab. Vinculin was used as internal control. (Fig 12A). Fig 12B shows that cell detachment (i.e. acantholysis) is prevented by anti-FasL Ab or caspase-8 inhibitor. These results indicate that inhibiting FasL or the caspase-8-activated apoptotic pathway prevents both caspase-8 activation and dsg cleavage, thus blocking acantholysis.

In conclusion we have shown that FasL exert dual activity, by both activating the caspase-8 mediated extrinsic apoptotic pathway and Dsg cleavage. In agreement with our work, Wang and coworkers (Wang et al, 2004) have suggested that apoptosis could be the cause of the acantholytic phenomenon. They showed that PV-IgG and an antibody against Fas receptor (anti-FasR) induce lesions in vitro in a similar way, causing: (1) secretion of soluble FasL; (2) elevated cellular amounts of FasR, FasL (soluble and membranal), Bax and p53 proteins; (3) reduction in levels of cellular Bcl-2; (4) enrichment in caspase 8, and activation of caspases 1 and 3; (5) coaggregation of FasL and FasR with caspase 8 in membranal death-inducing signaling complex (DISC). Hence, the Fas-mediated death signaling pathway seems to be involved in lesion formation.

A well established animal model has been long and widely used for studying pemphigus. Passive transfer of PVIgG into neonatal mice induce cell detachment and the formation of the bulla. This model has been used to assess the involvement of apoptosis and FasL in the pathogenesis of pemphigus. We injected subcutaneously PVIgG (5mg/g/BW) purified from patients sera in newborn C57BL/6N Crl mice. Normal newborn mice treated with IgG purified from sera of healthy individuals (NIgG) will be used as controls. Animals were sacrificed 20 hours after injection.

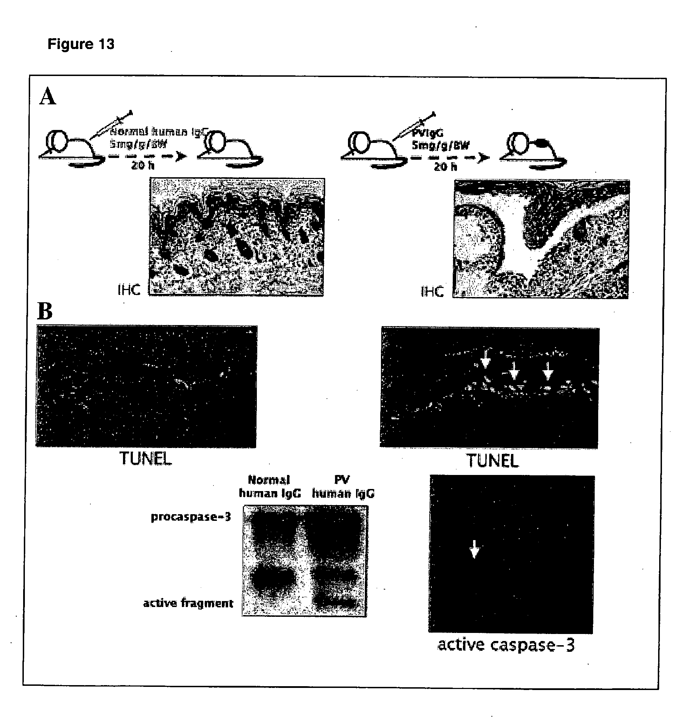

Hematoxilin and eosin staining shows that blister develop only in mice treated with PVIgG, but not in mice treated with normal human IgG (Fig 13A). Apoptosis was detected either by TUNEL or by caspase-3 activation only in mice treated with PVIgG (Fig 13B).

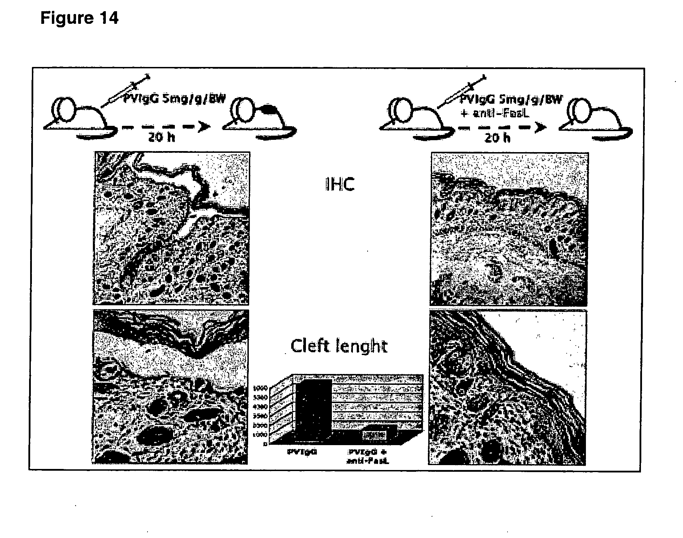

In order to evaluate the role of FasL in vivo, mice were treated with PVIgG or PVIgG plus anti-FasL antibody (MFL3 clone, specific for mouse). Anti-FasL (40 µg/mouse) was administered 3 hrs after PVIgG injection and prevented blister formation in mice, as shown by H & E staining. In addition, the lenght of clefts in anti-FasL treated mice was markedly reduced (Fig 14).

In conclusion we have shown that FasL exert dual activity, by both activating the caspase-8-mediated extrinsic apoptotic pathway and Dsg cleavage. Most importantly, blocking FasL protects from acantholysis in vitro and in vivo.

### References

Aoudjit F and Vuori K: Matrix attachment regulates FAS-induced apoptosis in endothelial cells: a role for c-FLIP and implications for anoikis. J Cell Biol 152: 633-643, 2001
Arredondo J et al Am J Pathol 167:1531-1544, 2005
Bahr GM, Capron A, Dewulf J, Nagata S, Tanaka M, Bourez JM, Mouton Y. Elevated serum level of Fas ligand correlates with the asymptomatic stage of human immunodeficiency virus infection. Blood 90:896-898, 1997
Bergin E, Levine JS, Koh JS, Lieberthal W: Mouse proximal tubular cell-cell adhesion inhibits apoptosis by a cadherin-dependent mechanism. Am J Physiol Renal Physiol 278: F758-F768, 2000
Du, Z., et al., Biochem. Biophys. Res. Commun., 1996 226, 595-600
Frisch SM: Evidence for a function of death-receptor-related, death-domain-containing proteins in anoikis. Current Biol 9:1047-1049, 1999
Frisch SM, Francis H: Disruption of epithelial cell-matrix interactions induces apoptosis. J Cell Biol 124:619-626, 1994
Frisch SM, Screaton RA: Anoikis mechanisms. Curr Opin Cell Biol 13:555-562, 2001
Giancotti FG, Ruoslahti E: Integrin signaling. Science 285: 1028-1032, 1999
Gniadecki R, Jemec GBE, Thomsen BM, Hansen M: Relationship between keratinocyte adhesion and death: anoikis in acantholytic diseases. Arch Dermatol Res 290:528-532, 1998
Grossman J, Walther K, Artinger M, Kiessling S, Scholmerich J: Apoptotic signaling during initiation of detachment-induced apoptosis ("anoikis") of primary human intestinal epithelial cells. Cell Growth & Diff 12:147-155, 2001
Jolles S, Hughes J, Whittaker S: Dermatological uses of high-dose intravenous immunoglobulin. Arch Dermatol 134:80-86, 1998
Juo P, Kuo CJ, Yuan J, Blenis J: Essential requirement for caspase-8/FLICE in the initiation of the Fas-induced apoptotic cascade. Curr Biol 8:1001-1008, 1998
Kalish RS: pemphigus vulgaris: the other half of the story. J Clin Invest 106: 1433-1435, 2000
Kitajima Y :Mechanisms of desmosome assembly and disassembly. Clin Exp Dermatol. 27:684-90, 2002
Kovacs B, Liossis SN, Dennis GJ, Tsokos GC: Increased expression of functional Fas ligand in activated T-cells from patients with systemic lupus erythematosus. Autoimmunity 25:213-221, 1997
Lee, J., et al. Endocrinology, 1997, 138, 2081-2088
Li N, Zhao M, Liu Z, Diaz LA: Pathogenic role of apoptosis in experimental pemphigus. J Invest Dermatol 126: 173 Abstract, 2006
Marconi A, Atzei P, Panza C, Fila C, Tiberio R, Truzzi F, Wachter T, Leverkus M, Pincelli C: FLICE/caspase-8 activation triggers anoikis induced by β1 integrin blockade in human keratinocytes. J Cell Sci 117: 5815-5823, 2004
Marconi A, Vaschieri C, Zanoli S, Giannetti A, Pincelli C Nerve growth factor protects human keratinocytes from ultraviolet-B-induced apoptosis. J Invest Dermatol 113:920-927, 1999
Nishimura S, Adachi M, Ishida T, Matsunaga T, Uchida H, Hamada H, Imai K: Adenovirus-mediated transfection of caspase-8 augments anoikis and inhibits peritoneal dissemination of human gastric carcinoma cells. Cancer Res 61:7009-7014, 2001
O'Connell, J., et al. J. Exp. Med., , 184, 1075-1082, 1996
Payne AS Curr Opin Cell Biol 16: 536-543, 2004
Pincelli C, Haake AR, Benassi L, Grassilli E, Magnoni C, Ottani D, Polakowska R, Franceschi C, Giannetti A Autocrine nerve growth factor protects human keratinocytes from apoptosis through its high affinity receptor (trk): a role for bcl-2. J Invest Dermatol 109: 757-764, 1997
Queen et al., Proc. Natl. Acad. Sci. USA 86 (1989), 10029, and Verhoeyan et al., Science 239 (1988), 1534
Reed JC: Mechanisms of apoptosis. Am J Pathol 157:1415-1430
Rezgui SS, Honore S, Rognoni J-B, Martin P-M, Penel C: Up-regulation of α2β1 integrin cell-surface expression protects A431 cells from epidermal growth factor-induced apoptosis. Int J Cancer 87: 360-367, 2000
Rytomaa M, Martins LM, Downward J: Involvement of FADD and caspase-8 signalling in detachment-induced apoptosis. Current Biol 9:1043-1046, 1999
Sharma K, Wang RX, Zhang LY, Yin DL, Luo XY, Solomon JC, Jiang RF, Markos K, Davidson W, Scott DW, Shi YF: Death the Fas way: regulation and pathophysiology of Fas and its ligand. Pharmacol Ther. 88:333-347, 2000
Tiberio R, Marconi A, Fila C, Fumelli C, Pignatti M, Krajewski S, Giannetti A, Reed JC, Pincelli C: Keratinocytes enriched for stem cells are protected from anoikis via an integrin signaling pathway, in a Bcl-2 dependent manner. FEBS Letters 26318:1-6, 2002
Turley, J. M., et al. Cancer Res., 1997, 57, 881-890
Viard I, Wehrli P, Bullani R, Schneider P, Holler N, Salomon D, Hunzinker T, Saurat J-H, Tschopp J, French L: Inhibition of toxic epidermal necrolysis by blockade of Fas with human intravenous immunoglobulin. Science 282:490-493, 1998
Wang X. et al: Possible apoptotic mechanism in epidermal cell acantholysis induced by pemphigus vulgaris autoimmunoglobulins Apoptosis 2004; 9: 131-143
Wehrli P, Viard I, Bullani R, Tschopp J, French LE. Death receptors in cutaneous biology and disease. J Invest Dermatol 115:141-148, 2000
Weiske J, Schoneberg T, Schroder W, Hatzfeld M, Tauber R, Huber O: The fate of desmosomal proteins in apoptotic cells. J Biol Chem 276:41175-41181, 2001
Yang, Y., et al., J. Exp. Med., 1995, 181, 1673-1682
Yu, W. et al. Cancer Res., 1999, 59, 953-961

## Claims

1. Antibody selected from:
(i) a monoclonal antibody or an antigen-binding fragment thereof specific for human Fas ligand protein (FasL), wherein said monoclonal antibody comprises at least one heavy chain variable region and at least one light chain variable region, wherein the amino acid sequences of the complementary determining regions (CDRs) of the heavy chain are:
(a₁) CDR H1: Asn Tyr Trp Ile Gly (SEQ ID NO:1),
(b₁) CDR H2: Tyr Leu Tyr Pro Gly Gly Leu Tyr Thr Asn Tyr Asn Glu Lys Phe Lys Gly (SEQ ID NO: 2),
(c₁) CDR H3: Tyr Arg Asp Tyr Asp Tyr Ala Met Asp Tyr (SEQ ID NO: 3)
and the amino acid sequences of the complementary determining regions (CDRs) of the light chain are
(a₂) CDR L1: Lys Ser Thr Lys Ser Leu Leu Asn Ser Asp Gly Phe Thr Tyr Leu Gly (SEQ ID NO: 4),
(b₂) CDR L2: Leu Val Ser Asn Arg Phe Ser (SEQ ID NO: 5),
(c₂) CDR L3: Phe Gln Ser Asn Tyr Leu Pro Leu Thr (SEQ ID NO: 6) for use in the prevention and/or treatment of pemphigus.

2. Antibody selected from
(i) a monoclonal antibody or an antigen-binding fragment thereof specific for human Fas ligand protein (FasL), wherein said monoclonal antibody comprises at least one heavy chain variable region and at least one light chain variable region, wherein the amino acid sequences of the complementary determining region (CDRs) of the heavy chain are:
(a₁) CDR H1: Glu Tyr Pro Met His (SEQ ID NO: 7),
(b₁) CDR H2: Met Ile Tyr Thr Asp Thr Gly Glu Pro Ser Tyr Ala Glu Glu Phe Lys Gly (SEQ ID NO: 8),
(c₁) CDR H3: Phe Tyr Trp Asp Tyr Phe Asp Tyr (SEQ ID NO: 9) and the amino acid sequences of the complementary determining regions (CDRs) of the light chain are
(a₂) CDR L1: Arg Ala Ser Gln Asp Ile Ser Asn Tyr Leu Asn (SEQ ID NO: 10),
(b₂) CDR L2: Tyr Thr Ser Arg Leu His Ser (SEQ ID NO: 11),
(c₂) CDR L3: Gln Gln Gly Ser Thr Leu Pro Trp Thr (SEQ ID NO: 12) for use in the prevention and/or treatment of pemphigus.

3. The antibody for use according to any one of claims 1 or 2, wherein the antibody or an antigen-binding fragment thereof is selected from a partially or fully humanized antibody, a partially or fully humanized single chain antibody or a fragment thereof.

4. The antibody for use according to any one of the preceding claims in a combination therapy with at least one further therapy effective against pemphigus.

5. The antibody for use according to claim 4 in combination with at least one further immuno-suppressive drug, in particular with at least one further steroid.

## Patentansprüche

1. Antikörper ausgewählt aus:
(i) einem monoklonalen Antikörper oder einem Antigen-bindenden Fragment davon spezifisch für humanes Fas Ligand Protein (FasL), wobei der monoklonale Antikörper mindestens eine schwere Kette variable Region und mindestens eine leicht Kette variable Region umfasst, wobei die Aminosäure-Sequenzen der Komplementaritäts-bestimmenden Regionen (complementary determining regions CDRs) der schweren Kette sind:
(a₁) CDR H1: Asn Tyr Trp Ile Gly (SEQ ID NO:1),
(b₁) CDR H2: Tyr Leu Tyr Pro Gly Gly Leu Tyr Thr Asn Tyr Asn Glu Lys Phe Lys Gly (SEQ ID NO:2),
(c₁) CDR H3: Tyr Arg Asp Tyr Asp Tyr Ala Met Asp Tyr (SEQ ID NO:3)
und die Aminosäuresequenzen der Komplementarität-bestimmenden Regionen (complementary determining regions CDRs) der leichten Kette sind:
(a₂) CDR L1: Lys Ser Thr Lys Ser Leu Leu Asn Ser Asp Gly Phe Thr Tyr Leu Gly (SEQ ID NO:4),
(b₂) CDR L2: Leu Val Ser Asn Arg Phe Ser (SEQ ID NO:5),
(c₂) CDR L3: Phe Gln Ser Asn Tyr Leu Pro Leu Thr (SEQ ID NO:6) zur Verwendung in der Prävention und/oder Behandlung von Pemphigus.

2. Antikörper ausgewählt aus:
(i) einem monoklonalen Antikörper oder einem Antigen-bindenden Fragment davon spezifisch für humanes Fas Ligand Protein (FasL), wobei der monoklonale Antikörper mindestens eine schwere Kette variable Region und mindestens eine leicht Kette variable Region umfasst, wobei die Aminosäure-Sequenzen der Komplementaritäts-bestimmenden Regionen (complementary determining regions CDRs) der schweren Kette sind:
(a₁) CDR H1: Glu Tyr Pro Met His (SEQ ID NO:7),
(b₁) CDR H2: Met Ile Tyr Thr Asp Thr Gly Glu Pro Ser Tyr Ala Glu Glu Phe Lys Gly (SEQ ID NO:8),
(c₁) CDR H3: Phe Tyr Trp Asp Tyr Phe Asp Tyr (SEQ ID NO:9)
und die Aminosäuresequenzen der Komplementarität-bestimmenden Regionen (complementary determining regions CDRs) der leichten Kette sind:
(a₂) CDR L1: Arg Ala Ser Gln Asp Ile Ser Asn Tyr Leu Asn (SEQ ID NO:10),
(b₂) CDR L2: Tyr Thr Ser Arg Leu His Ser (SEQ ID NO:11),
(c₂) CDR L3: Gln Gln Gly Ser Thr Leu Pro Trp Thr (SEQ ID NO:12) zur Verwendung in der Prävention und/oder Behandlung von Pemphigus.

3. Antikörper zur Verwendung nach einem der Ansprüche 1 oder 2, wobei der Antikörper oder das Antigen-bindende Fragment davon ausgewählt ist aus einem teilweise oder vollständig humanisiertem Antikörper, einem teilweise oder vollständig humanisiertem Einzel-Ketten Antikörper oder einem Fragment davon.

4. Antikörper zur Verwendung nach einem der vorhergehenden Ansprüche in einer Kombinationstherapie mit mindestens einer weiteren Therapie, die effektiv gegen Pemphigus ist.

5. Antikörper zur Verwendung nach Anspruch 4 in Kombination mit mindestens einem weiteren immun-suppressiven Arzneimittel, insbesondere mit mindestens einem weiteren Steroid.

## Revendications

1. Anticorps choisi parmi :
(i) un anticorps monoclonal ou un fragment liant l'antigène de celui-ci, spécifique de la protéine ligand Fas humaine (FasL), où ledit anticorps monoclonal comprend au moins une région variable de chaine lourde et au moins une région variable de chaine légère, où les séquences des acides aminés des régions déterminant la complémentarité (CDR) de la chaine lourde sont :
(a₁) CDR H1 : Asn Tyr Trp Ile Gly (SEQ ID N°1),
(b₁) CDR H2 : Tyr Leu Tyr Pro Gly Gly Leu Tyr Thr Asn Tyr Asn Glu Lys Phe Lys Gly (SEQ ID N°2),
(c₁) CDR H3 : Tyr Arg Asp Tyr Asp Tyr Ala Met Asp Tyr (SEQ ID N°3),
et les séquences des acides aminés des régions déterminant la complémentarité (CDR) de la chaine légère sont :
(a₂) CDR L1 : Lys Ser Thr Lys Ser Leu Leu Asn Ser Asp Gly Phe Thr Tyr Leu Gly (SEQ ID N°4),
(b₂) CDR L2 : Leu Val Ser Asn Arg Phe Ser (SEQ ID N°5),
(c₂) CDR L3 : Phe Gln Ser Asn Tyr Leu Pro Leu Thr (SEQ ID N°6),
à utiliser dans la prévention et/ou le traitement du pemphigus.

2. Anticorps choisi parmi :
(i) un anticorps monoclonal ou un fragment liant l'antigène de celui-ci, spécifique de la protéine ligand Fas humaine (FasL), où ledit anticorps monoclonal comprend au moins une région variable de chaine lourde et au moins une région variable de chaine légère, où les séquences des acides aminés des régions déterminant la complémentarité (CDR) de la chaine lourde sont :
(a₁) CDR H1 : Glu Tyr Pro Met His (SEQ ID N°7),
(b₁) CDR H2 : Met Ile Tyr Thr Asp Thr Gly Glu Pro Ser Tyr Ala Glu Glu Phe Lys Gly (SEQ ID N°8),
(c₁) CDR H3 : Phe Tyr Trp Asp Tyr Phe Asp Tyr (SEQ ID N°9),
et les séquences des acides aminés des régions déterminant la complémentarité (CDR) de la chaine légère sont :
(a₂) CDR L1 : Arg Ala Ser Gln Asp Ile Ser Asn Tyr Leu Asn (SEQ ID N°10),
(b₂) CDR L2 : Tyr Thr Ser Arg Leu His Ser (SEQ ID N°11),
(c₂) CDR L3 : Gln Gln Gly Ser Thr Leu Pro Trp Thr (SEQ ID N°12),
à utiliser dans la prévention et/ou le traitement du pemphigus.

3. Anticorps à utiliser selon l'une quelconque des revendications 1 ou 2, où l'anticorps ou un fragment liant l'antigène de celui-ci est choisi parmi un anticorps partiellement ou complètement humanisé, un anticorps à chaine unique partiellement ou complètement humanisé, ou un fragment de ceux-ci.

4. Anticorps à utiliser selon l'une quelconque des revendications précédentes, dans une thérapie combinée avec au moins un autre traitement efficace contre le pemphigus.

5. Anticorps à utiliser selon la revendication 4, en combinaison avec au moins un médicament immunosuppresseur, en particulier avec au moins un stéroïde supplémentaire.
